# EUROPÄISCHE PATENTANMELDUNG

(11) **EP 2 181 643 A1**
(43) Veröffentlichungstag der Anmeldung: **05.05.2010**
(21) Anmeldenummer: 08019005.1
(22) Anmeldetag: 30.10.2008
(51) Int. Cl.: A61B 1/247, A61B 1/253

(54) **Instrument für zahnmedizinische Zwecke mit einem Spiegel**

(71) Anmelder: Dental Care Nordic AB, 57002 Stockaryd (SE)
(72) Erfinder: Senghaas, Thomas, 22085 Hamburg (DE); Hultqvist, Torbjörn, 57002 Stockaryd (SE); Clasén, Kristina, 57332 Tranas (SE); Asterhed, Tommy, 57635 Sävsjö (SE); Svensson, Lars, 56146 Huksvarna (SE); Steinhoff, Frank, 20099 Hamburg (DE)
(74) Vertreter: Glawe, Delfs, Moll

(57) **Zusammenfassung**

Das Instrument für zahnmedizinische Untersuchungen und Behandlungen mit einem Spiegel (2), der mit einer Achse (9) drehbar in einem einen Handgriff aufweisenden Halter (10) gelagert ist und über ein damit verbundenes Flügel- oder Turbinenrad (3) mit einer Unterdruckluftströmung drehend antreibbar ist, und mit einem Anschluss für eine Saugeinrichtung für die Unterdruckluftströmung, **dadurch gekennzeichnet, dass** das Flügel- oder Turbinenrad (3) in einer Kammer angeordnet ist, die durch ein scheibenförmiges Element (11) im Wesentlichen dichtend abgeschlossen ist, durch das die Achse (9) hindurchgeführt ist, und dass sie eine Zuluftleitung zur Kammer aufweist, die an einer vom Spiegel (2) entfernten Stelle eine oder mehrere nach außen führende Öffnungen aufweist.

## Beschreibung

Die Erfindung betrifft ein Instrument für zahnmedizinische Untersuchungen und Behandlungen mit einem Spiegel, der mit einer Achse drehbar in einem einen Handgriff aufweisenden Halter gelagert ist und über ein damit verbundenes Flügel- oder Turbinenrad mit einer Unterdruckluftströmung drehend antreibbar ist, und mit einem Anschluss für eine Saugeinrichtung für die Unterdruckluftströmung.

Sowohl der Zahnarzt/die Zahnärztin bzw. der Dentalhygieniker/die Dentalhygienikerin benötigen bei ihrer Arbeit einen Spiegel, da eine direkte Beobachtung des Zahns bzw. des zu behandelnden Teil des Zahns in vielen Fällen nicht möglich ist. Dieses Problem tritt einerseits bei "zweihändiger" Behandlung auf, wo der Zahnarzt bzw. die Dentalhygienikerin alleine arbeitet und dann auch den Spiegel halten muss. Aber auch bei "vierhändiger" Behandlung, wenn dem/der Behandelnden noch ein Assistent oder eine Assistentin zur Verfügung steht, müssen solche Spiegel verwendet werden. Auf diesen Spiegeln setzt sich leicht Speichel, Schleim oder Blut ab, so dass eine Beobachtung mit Hilfe des Spiegels nicht mehr möglich ist. Dieses Problem wird noch größer, wenn mit flüssigkeitsgekühlten Bohrern oder Ähnlichem gearbeitet wird und das versprühte Kühlwasser sich auf dem Spiegel absetzt. Außerdem können sich Schleifteilchen oder bei der Zahnreinigung entfernter Zahnstein auf dem Spiegel absetzen. All dies erfordert häufiges Herausnehmen des Spiegels aus dem Mund des Patienten und Reinigen desselben und somit eine ständige Arbeitsunterbrechung des Workflows.

Um diese Probleme zu beseitigen, ist es bekannt, den Spiegel drehbar anzuordnen und mit einem Elektromotor in schnelle Drehung zu versetzen (US 6,575,744 B1). Sich auf dem Spiegel absetzende Flüssigkeiten und feste Teilchen werden bei entsprechender Drehzahl, die höher sein sollte als ungefähr 1000 Drehungen pro Minute, aufgrund der Fliehkraft vom Spiegel entfernt. Selbst wenn Reste auf dem Spiegel zurückbleiben, stören diese weniger, da wegen der schnellen Rotation des Spiegels das störende Bild dieser Teilchen ausgemittelt wird. Der elektrische Antrieb mittels eines Motors und der entsprechenden Stromversorgung ist aber aufwendig, teuer und erhöht das Gewicht des in der Hand zu haltenden Dentalinstruments. Außerdem bringt die Sterilisierung eines solchen Instruments mit darin eingebautem Motor erhebliche Probleme mit sich, falls sie überhaupt möglich ist, ohne den Motor zu beschädigen oder zu zerstören.

Diese Probleme werden teilweise bei einem Instrument der eingangs genannten Art vermieden. Hier wird der Spiegel mit Hilfe einer Unterdruck-Luftströmung in Drehung versetzt. Der Spiegel ist am Rand mit einem Flügel- oder Turbinenrad versehen. Unterhalb des Spiegels befindet sich ein Hohlraum, der an die Saugleitung einer Saugeinrichtung angeschlossen wird, die in jeder Zahnarztpraxis zum Absaugen von Speichel, Schleim und Blut vorgesehen ist (SE 507193 C2). Das entsprechende Instrument ist leichter und kleiner, da ein Motor und Batterien nicht vorgesehen sind. Es ist kostengünstiger herzustellen und auch ohne große Schwierigkeiten sterilisierbar. Es hat auch den Vorteil, dass es gleichzeitig als Absauger wirkt, da nicht nur Luft am Rand des Spiegels eingesaugt wird und den Spiegel in Drehung versetzt, sondern auch Flüssigkeiten und Festkörperteilchen eingesaugt werden, die sich im Munde des Patienten befinden. Dies ist aber gleichzeitig ein schwerwiegender Nachteil, da solche Festkörperteilchen (z. B. Zahnsteinteilchen) sich im Spalt zwischen Spiegel und Gehäuse festsetzen können und so die Drehung des Spiegels behindern oder sogar unmöglich machen können. Ein weiterer Nachteil besteht darin, dass der Unterdruck, der in der Kammer unterhalb des Spiegels herrscht, voll auf den Spiegel wirkt, so dass dieser mit einer Kraft in Richtung auf die Kammer beaufschlagt wird, was beträchtliche Lagerkräfte bzw. Verschleiß des Lagers mit sich bringt.

Eine Belastung des Spiegels mit einer Druckdifferenz tritt auch bei einem geschlossenen System auf, bei dem im Bereich des Spiegels kein Luftaustausch mit der Umgebung stattfindet (US 4,408,991). Dort wird Überdruck in eine Kammer eingeblasen und aus dieser wieder abgelassen, was den Spiegel zwar in Drehung versetzt, diesen aber mit einem Druck beaufschlagt, der durch ein Gleitlager am Rand des Spiegels aufgenommen werden muss.

Die Aufgabe der Erfindung besteht in der Schaffung einer Vorrichtung der eingangsgenannten Art, mit der problemlos und zuverlässig der Spiegel frei von Verunreinigungen gehalten werden kann.

Die erfindungsgemäße Lösung besteht darin, dass das Flügel- oder Turbinenrad in einer Kammer angeordnet ist, die durch ein scheibenförmiges Element im Wesentlichen dichtend abgeschlossen ist, durch das die Achse hindurchgeführt ist, und dass sie eine Zuluftleitung zur Kammer aufweist, die an einer vom Spiegel entfernten Stelle eine oder mehrere nach außen führende Öffnungen aufweist.

Das Flügel- oder Turbinenrad ist wie beim Stand der Technik ebenfalls in einem mit Unterdruck beaufschlagten Raum untergebracht. Dieser Unterdruck wirkt aber im Gegensatz auf das vorbekannte Instrument nicht auf den Spiegel. Der Spiegel wird also praktisch kräftefrei gelagert, da er auf allen Seiten von Atmosphärendruck umgeben ist. Es können auch keine Flüssigkeiten oder Festteilchen in den Spalt zwischen Spiegel und umgebendem Gehäuse hineingesaugt werden, da der Unterdruck nur in der im Wesentlichen abgeschlossenen Kammer vorherrscht.

Schließlich besteht der weitere Vorteil, dass das Flügel- oder Turbinenrad nicht mit Luft beaufschlagt wird, die mit Flüssigkeit oder festen Teilchen belastet ist, da die Luft, die infolge des Unterdrucks angesaugt wird und das Flügel- oder Turbinenrad antreibt, nicht dem Mund des Patienten entnommen wird, sondern an Öffnungen einströmt, die außerhalb des Mundes des Patienten angeordnet sind.

Die Achse des Spiegels durchdringt das scheibenförmige Element, das als Trennscheibe wirkt. Ein weiterer Vorteil liegt in der leichten Entnahme der Spiegel-Trennscheiben-Turbineneinheit. Somit ist eine gesicherte hygienische Aufbereitung von Kammer und Turbine gewährleistet und auch der leichte Austausch von Verschleißteilen. Zweckmäßigerweise geschieht dies mit geringem Spiel, so dass die Achse nicht nur im Halter, sondern auch durch das scheibenförmige Element gelagert wird.

Die nach außen führenden Öffnungen könnten z. B. im Saugrohr angeordnet sein, das zu diesem Zweck zumindest im Endbereich zweilumig ausgebildet ist. Bei einer zweckmäßigen Ausführungsform sind diese Öffnungen aber in der Nähe des vom Spiegel entfernten Endes des Handgriffs angeordnet. Dabei sind zweckmäßigerweise mehrere Öffnungen vorgesehen und/oder diese mit Ausnehmungen oder Erhebungen versehen, so dass nicht alle Öffnungen gleichzeitig durch die Hand der Bedienungsperson geschlossen werden könnten, wodurch die rotierende Wirkung des Spiegels unterbrochen würde.

Ein solches Instrument ist insbesondere für den vierhändigen Betrieb geeignet, wo die notwendige Absaugung durch einen Assistenten stattfindet und der Zahnarzt den Spiegel in der linken und das Behandlungsinstrument in der rechten Hand halten kann. Das Instrument ist dann einfach aufgebaut und kann kostengünstig und mit besonders geringen Abmessungen hergestellt werden.

In anderen Fällen möchte man aber nicht nur eine Beobachtung mit dem Spiegel durchführen, sondern gleichzeitig absaugen. Dies ist insbesondere beim zweihändigen Betrieb der Fall, bei dem der Zahnarzt oder die Zahnhygienikerin alleine am Patienten arbeitet und gleichzeitig beobachten, absaugen und behandeln muss. In diesem Fall ist zweckmäßigerweise ein zusätzlicher Absaugkanal vorgesehen, der mit demselben Saugschlauch verbunden ist, und zweckmäßigerweise mündet dieser Kanal in der Nähe des Spiegels, da hier besonders gut abgesaugt werden muss. In vielen Fällen kann es wünschenswert sein, vorübergehend die Saugleistung zu erhöhen. Für diesen Zweck ist bei einer vorteilhaften Ausführung vorgesehen, das Verteilungsverhältnis der Saugleistung zwischen Saugöffnung und Turbinenantrieb mittels einer Drossel einstellbar zu gestalten. So kann in besonderen Behandlungssituationen der Schwerpunkt auf perfekte Sicht, oder optimale Saugwirkung gewählt werden.

Bei einer vorteilhaften Ausführungsform ist auch eine Beleuchtungseinrichtung vorgesehen, um die Behandlungsstelle zu beleuchten. Hier sind insbesondere eine oder mehrere Licht emittierende Dioden (LEDs) zweckmäßig, die sich durch geringe Wärmeentwicklung auszeichnen. Trotzdem kann es zweckmäßig sein, die entsprechende Anordnung so vorzusehen, dass die Licht emittierenden Dioden durch die Luftströmungen gekühlt werden.

Die Licht emittierenden Dioden können durch Batterien mit Strom versorgt werden, die im Instrument, insbesondere im Handgriff vorgesehen sind. Andererseits wäre eine elektrische Leitung in Verbindung mit dem Absaugschlauch denkbar. Bei einer anderen vorteilhaften Ausführungsform kann aber vorgesehen werden, dass das Instrument einen mit der Achse verbundenen Generator zur Energieversorgung der Beleuchtungseinrichtung aufweist.

Die Lichtquellen der Beleuchtungseinrichtung könnten auch außerhalb des Bereichs des Spiegels vorgesehen sein, wobei dann das Licht für die Beleuchtung im Bereich des Spiegels mit einem Lichtleiter von der Lichtquelle zugeführt wird.

Die Erfindung wird im Folgenden anhand von vorteilhaften Ausführungsformen unter Bezugnahme auf die beigefügten Zeichnungen beispielsweise beschrieben es zeigen:
- Fig. 1: teilweise weggeschnitten eine erste Ausführungsform des erfindungsgemäßen Instruments;
- Fig. 2: eine Detaildarstellung der Lagerung und des Antriebs des Spiegels; und
- Fig. 3: eine perspektivische Ansicht einer anderen Ausführungsform.

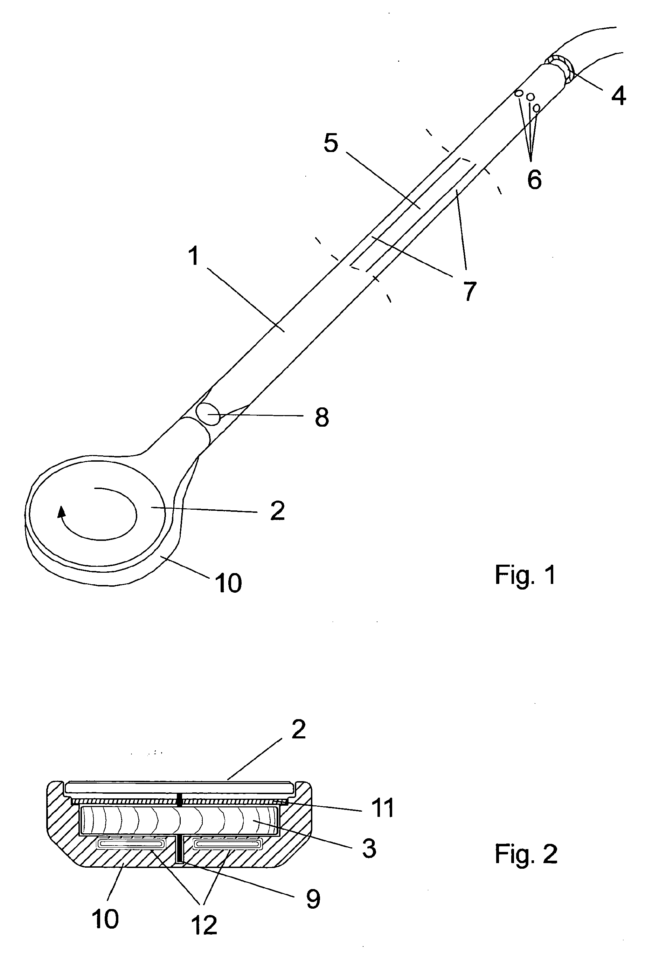

Bei der in Fig. 1 gezeigten Ausführungsform besitzt das Instrument einen Handgriff 1, an dessen Ende ein Spiegel 2 drehbar angebracht ist. Dieser Spiegel wird dadurch in Drehung versetzt, dass unterhalb desselben ein Flügelrad 3 angeordnet ist, das in Fig. 2 gezeigt ist. An einen Schlauchverbinder 4 wird ein Saugschlauch angesetzt, der durch einen Kanal 5 Luft aus dem Bereich unterhalb des Spiegels 3 ansaugt. Luft von außen strömt durch Öffnungen 6 durch einen zweiten Kanal 7 in diesen Bereich nach. In der Fig. 1 ist dabei noch eine Beleuchtungseinrichtung in Form einer Diode 8 dargestellt.

Fig. 2 zeigt im Querschnitt den Spiegel 2 mit seinem Antrieb. Der Spiegel 2 ist mit einer Achse 9, die im Gehäuse 10 gelagert ist, mit einem Flügel- oder Turbinenrad 3 verbunden. Zwischen Spiegel 2 und Flügel- oder Turbinenrad 3 befindet sich eine Scheibe 11, die in wesentlichen dichtend am Gehäuse 10 anliegt und durch die die Achse 9 hindurchgeführt ist. Der Unterdruck, der durch den Kanal 5 angelegt wird, wirkt daher nur im Bereich des Flügelrades 3 bzw. in der entsprechenden Kammer, nicht aber auf den Spiegel 2. Bei der Lagerung des Spiegels müssen daher keine Druckkräfte aufgenommen werden.

Die ganze Anordnung ist einfach aufgebaut und auch ohne weiteres sterilisierbar. Wenn ein geeigneter Generator 12 zur Verfügung steht, der bei der Sterilisierung keine Probleme bereitet, so könnte dieser mit der Achse 9 verbunden sein, wie dies in Fig. 2 schematisch dargestellt ist. Mit Hilfe dieses Generators 12 könnte dann die Energie zum Betrieb der LED 8 erzeugt werden.

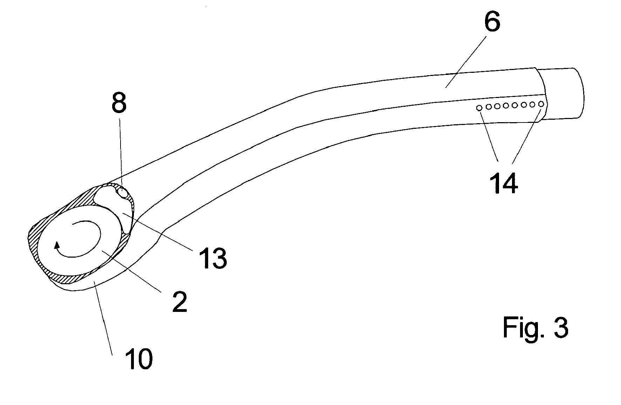

Bei der Ausführungsform der Fig. 3 ist zusätzlich eine Absaugleitung 13 vorgesehen, die sich im Bereich der LED 8 und des Spiegels 2 öffnet. Mit 14 sind Vorsprünge in der Nähe der Lufteintrittsöffnungen 6 bezeichnet, durch die verhindert werden soll, dass durch di Hand der Bedienungsperson gleichzeitig alle Öffnungen 6 verschlossen werden. Die Saugwirkung sowohl für die Antrieb des Spiegels 2 als auch für die Absaugung bei 13 kann noch durch einen in den Fig. nicht gezeigte Drossel verringert bzw. eingestellt werden.

## Patentansprüche

1. Instrument für zahnmedizinische Untersuchungen und Behandlungen mit einem Spiegel (2), der mit einer Achse (9) drehbar in einem einen Handgriff (1) aufweisenden Halter (10) gelagert ist und über ein damit verbundenes Flügel- oder Turbinenrad (3) mit einer Unterdruckluftströmung drehend antreibbar ist, und mit einem Anschluss (4) für eine Saugeinrichtung für die Unterdruckluftströmung, **dadurch gekennzeichnet, dass** das Flügel- oder Turbinenrad (3) in einer Kammer angeordnet ist, die durch ein scheibenförmiges Element (11) im Wesentlichen dichtend abgeschlossen ist, durch das die Achse (9) hindurchgeführt ist, und dass sie eine Zuluftleitung (7) zur Kammer aufweist, die an einer vom Spiegel (2) entfernten Stelle eine oder mehrere nach außen führende Öffnungen (6) aufweist.

2. Instrument nach Anspruch 1, **dadurch gekennzeichnet, dass** die Achse (9) im scheibenförmigen Element (11) gelagert ist.

3. Instrument nach Anspruch 1 oder 2, **dadurch gekennzeichnet, dass** die nach außen führende(n) Öffnung(en) (6) der Zuluftleitung (7) in der Nähe des vom Spiegel (2) entfernten Ende des Handgriffs (1) angeordnet ist/sind.

4. Instrument nach Anspruch 3, **dadurch gekennzeichnet, dass** die nach außen führenden Öffnung(en) (6) der Zuluftleitung (7) an Stellen des Handgriffs (1) angeordnet bezw. mit Vorsprüngen/Ausnehmungen (14) versehen ist/sind, die ein Verschließen der Öffnungen (6) mit der Hand zumindest erschweren.

5. Instrument nach einem der Ansprüche 1 bis 4, **dadurch gekennzeichnet, dass** es eine zusätzliche Absaugleitung (13) aufweist, die mit der Saugeinrichtung verbunden ist und in der Nähe des Spiegels (2) mündet.

6. Instrument nach einem der Ansprüche 1 bis 5, **dadurch gekennzeichnet, dass** es mit Einrichtungen zum Drosseln der Unterdruckströmung(en) versehen ist.

7. Instrument nach einem der Ansprüche 1 bis 6, **dadurch gekennzeichnet, dass** es eine Beleuchtungseinrichtung, insbesondere eine oder mehrere Licht emittierende Dioden (LEDs 8) aufweist.

8. Instrument nach Anspruch 7, **dadurch gekennzeichnet, dass** es einen mit der Achse (9) verbundenen Generator (12) zur Energieversorgung der Beleuchtungseinrichtung (8) aufweist.
